# EUROPEAN PATENT APPLICATION

(11) **EP 4 273 552 A1**
(43) Date of publication of application: **08.11.2023**
(21) Application number: 21914661.0
(22) Date of filing: 30.12.2021
(51) Int. Cl.: G01N 35/00, G01N 33/48, G01N 33/50

(54) **IN-VITRO MEDICAL DIAGNOSTIC SYSTEM**

(30) Priority: 31.12.2020 CN 202011629633
(71) Applicant: Edan Instruments, Inc., Shenzhen, Guangdong 518122 (CN)
(72) Inventor: ZHOU, Chuanchuan, Shenzhen, Guangdong 518122 (CN); HUANG, Gaoxiang, Shenzhen, Guangdong 518122 (CN); ZHAO, Zhixiang, Shenzhen, Guangdong 518122 (CN)
(74) Representative: De Arpe Tejero, Manuel
(86) International application number: PCT/CN2021/143216
(87) International publication number: WO 2022/143931

(57) **Abstract**

An in-vitro medical diagnostic system, comprising a main machine (1), a removable test card (2) and a removable reagent kit (3), wherein the reagent kit (3) includes a calibrating solution needed in a process for calibrating the test card (2); the reagent kit (3) is clamped to the main machine (1), with only first power drive and valve control transmission being provided between the main machine (1) and the reagent kit (3); and the test card (2) is mounted on the main machine (1) during the entire test process, with only second power drive and an optical conduction path being provided between the main machine (1) and the test card (2). No flow path is present in the main machine (1) of the in-vitro diagnostic system, thus eliminating hidden medical dangers. Tests for blood gas, hemoglobin and derivatives thereof can be achieved in a single test card.

## Description

### TECHNICAL FIELD

The present disclosure relates to the field of in-vitro technologies, and more particularly, to an in-vitro medical diagnosis system.

### BACKGROUND

The determination of gas component in blood is very important in various scientific researches and practical applications. In the rescue of critical clinical medicine patients, rapid and continuous determination of the partial pressure of carbon dioxide in the blood is critical. Especially for mechanically ventilated patients, the partial pressure of carbon dioxide in the blood is a key index for judging respiratory state of the patients, and various parameters of the breathing machine are mainly set according to the partial pressure of carbon dioxide in the blood of the patients. At present, the most widely used blood gas component detector in medicine is a blood gas analyzer, but conventional blood gas analyzers have the defects that a large number of blood samples need to be collected, the detection is discontinuous, the detection result is lagged, and the like.

The traditional in-vitro blood gas detection is performed in a large, well-equipped test center. Although these conventional test centers can provide efficient and accurate testing of large-volume fluid samples, it is not possible to provide a direct result. A practitioner must collect the fluid samples and send them to the laboratory, then the fluid samples are processed by the laboratory, and finally, the results are conveyed to the patients. This traditional detection method causes a long and complex cycle of blood gas detection, such that it is difficult for patients to obtain timely diagnostic results, which is not conducive to timely diagnosis by medical staff and cannot provide patients with a good medical experience.

In addition, the conventional in-vitro diagnostic testing requires training laboratory technicians to perform the test, thereby ensuring the accuracy and reliability of the test. However, usage errors caused by personnel handling samples may lead to surface contamination, sample spillage, or damage to diagnostic devices which results in increased repair and maintenance costs.

Moreover, in the related art, in-vitro medical diagnostic systems often have complex structures, especially flow paths are inside of the hosts of the in-vitro medical diagnosis systems. If the in-vitro medical diagnosis systems are not maintained in time after long-term use, it is easy to cause leakage of the internal flow paths of the hosts of the in-vitro medical diagnosis systems, which not only affects the accuracy of in-vitro diagnosis, but also brings serious safety and health hazards.

Moreover, for the in-vitro diagnostic apparatus of the related art, if only one test card is used to detect the blood of the patient, only blood gas detection is completed, or only hemoglobin detection is completed. If the indexes of blood gas and hemoglobin of the blood of the patient are required to be detected simultaneously, it is often necessary to take two samples of the patient's blood. This is not a serious issue for healthy adults, however, various inconveniences may be brought to newborns or patients with less blood volume (e.g., anemia). In addition, even for healthy adults, if the indexes of blood gas and hemoglobin are required to be detected simultaneously, two test cards are needed, which may greatly increase the diagnosis cost of the patients and cause waste of resources.

Further, in the related art, it is often necessary to calibrate the sensors in the test card before using the test card. Therefore, in order to save cost, the calibration liquid configured for calibration is usually stored in the test card. However, the consequent problem is that if the calibration liquid is stored in the test card, high storage conditions and environmental requirements are required for the test card during transportation and storage to ensure that the calibration liquid is not affected by adverse environmental factors such as temperature and humidity, thereby preventing the calibration of the test card sensor from being affected caused by the affected calibration liquid inside the test card.

Therefore, it is necessary to design an in-vitro medical diagnosis system that has simple and compact structure, and is safe and reliable, ensure that no hidden danger of flow path leakage exists in the host in the in-vitro medical diagnosis system. Besides, it may ensure that the calibration during detection is accurate and reliable, ensure that the detection of blood gas index and hemoglobin index are completed in one test card. The more ideal condition is that the same blood sample is used to complete the detection of blood gas index and hemoglobin index in one test card. The requirements for the storage condition and environment during the transportation and storage of the test card are low, and using the test card to detect has precise and reliable accuracy.

### SUMMARY OF THE DISCLOSURE

In view of the foregoing, a purpose of some embodiments of the present disclosure is to provide an in-vitro medical diagnostic device, and the in-vitro medical diagnostic device is capable of at least partially mitigating or eliminating at least one defect in the related art.

An in-vitro medical diagnostic device includes a housing including a first mounting area and a second mounting area, an optical system, and a power drive system. The first mounting area is configured to dispose a removable reagent pack, and the second mounting area is configured to dispose a removable test card. The optical system is disposed in the housing and configured for in-vitro diagnosis. The power drive system is disposed in the housing and configured to control at least pipelines in the removable test card to be fluidly connected to each other or occluded from each other, and/or control liquid flow in the removable reagent pack. The optical system is at least configured to detect a blood gas, a hemoglobin and/or an electrolyte in the test card.

In some embodiments, the in-vitro medical diagnostic device further includes a positioning connection area configured to connect an interface of the removable reagent pack and an interface of the removable test card.

In some embodiments, the optical system has one or more light source systems.

In some embodiments, at least one first light source system configured to activate a photochemical sensor in the removable test card is disposed below the second mounting area.

In some embodiments, the first light source system is configured to emit an excitation light, and the excitation light is configured to pass through a first optical path and enters one end of an optical fiber bundle, and the other end of the optical fiber bundle is provided with an optical element configured to focus and illuminate the photochemical sensor in the removable test card.

In some embodiments, the fiber optic bundle is configured to receive fluorescence and/or reflected light generated in the removable test card, and transmit the fluorescence and/or the reflected light to an optical detection element.

In some embodiments, the optical system further includes at least one second light source system disposed above the second mounting area and configured to detect hemoglobin and its derivatives and/or detect a flow position of internal liquid in the removable test card.

In some embodiments, an excitation light emitted from the first light source has a central wavelength of 450 nm to 470 nm.

In some embodiments, a central wavelength of the fluorescent and/or reflected light ranges from 520 nm to 650 nm.

In some embodiments, the optical system includes at least two light sources located on different sides of the second mounting area.

In some embodiments, the power drive system further includes a first power component configured to control the pipelines in the removable test card to be fluidly connected to each other or occluded from each other.

In some embodiments, the power drive system further includes a second power component configured to control switching of the pipelines and liquid flow in the removable reagent pack.

In some embodiments, the second power component includes a first motor configured to control switching of a three-way valve in the removable reagent pack and a second motor configured to control rotation of a peristaltic pump in the removable reagent pack.

In some embodiments, the power drive system is configured to output power in one or more modes of mechanical transmission, electricity, magnetism, or light.

In some embodiments, the in-vitro medical diagnostic device further includes one or more of: a display element configured to display diagnostic results, a printing element configured to output the diagnostic results, bar code/two-dimensional code reading elements, a battery, a UPS power supply.

An in-vitro medical diagnosis system includes a blood gas analyzer host, a removable test card, a removable reagent pack, and a power drive system. An interface of the removable reagent pack is fluidly connected to an interface of the removable test card, and an in-vitro medical diagnostic device is configured to provide power to the removable reagent pack and control pipelines of the removable test card to be fluidly connected to each other or occluded from each other, to complete calibration, sample detection and emptying.

In some embodiments, the removable test card includes at least a detection area in which blood gas is capable of being detected and a detection area in which hemoglobin and its derivatives are capable of being detected.

In some embodiments, a detection in the detection area in which hemoglobin and its derivatives are capable of being detected is implemented by colorimetric, electrochemical, or hemolytic method.

In some embodiments, the removable reagent pack includes at least one liquid storage device and its output pipelines, at least one transport control device configured to control at least flow directions of pipelines inside of the removable reagent pack, and at least one positioning mechanism configured to enable the transport control device to be driven by the in-vitro medical diagnostic device.

In some embodiments, pipelines between the detection area in which blood gas is capable of being detected and the detection area in which hemoglobin and its derivatives are capable of being detected are configured to be fluidly connected to each other or occluded from each other.

In some embodiments, the in-vitro medical diagnostic device is configured to control the pipelines of the removable test card to be fluidly connected to each other or occluded from each other, so that at least blood gas detection and hemoglobin detection is performed on the same blood sample or on different blood samples.

In some embodiments, the removable reagent pack at least includes calibration liquid required for calibration of the removable test card one or more times.

In some embodiments, an elastic material and/or a valve is disposed inside the removable test card or on a surface of the removable test card, and the elastic material and/or the valve is configured to allow the pipelines to be fluidly connected to each other or occluded from each other at a corresponding position of the removable test card.

In some embodiments, the removable test card includes at least three external interfaces; and the at least three external interfaces include a first interface configured to inject a calibration liquid, a second interface configured to inject a sample, and a third interface configured to be fluidly connected to a waste liquid area of the removable test card.

In some embodiments, the removable test card includes at least three pipeline control components.

In some embodiments, the at least three pipeline control components include a first pipeline control component, a second pipeline control component, and a third pipeline control component. The first pipeline control component is configured to control pipelines between the detection area in which blood gas is capable of being detected and the detection area in which hemoglobin and its derivatives are capable of being detected to be fluidly connected to each other or occluded from each other. The second pipeline control component is configured to enable a flow path inside of the removable test card to be fluidly connected to a sample inlet of the removable test card, or control the flow path inside of the removable test card to be occluded from the sample inlet of the removable test card. The third pipeline control component is configured to enable the waste liquid area of the removable test card to be fluidly connected to the flow path inside of the removable test card, or control the waste liquid area of the removable test card to be occluded from the flow path inside of the removable test card.

In some embodiments, the in-vitro medical diagnostic device is electrically connected to the removable test card.

In some embodiments, the in-vitro medical diagnostic device is electrically connected to the removable test card and configured to supply power to an electrochemical sensor in the removable test card by the power drive system.

The effects of the present disclosure are as follows. There is no flow path in the host of the in-vitro diagnostic system, therefore, there is no hidden danger of flow path leakage. Further, there is no calibration liquid in the test card, which reduces the requirements for the storage condition and environment of the test card in the transportation and storage links of the test card. Moreover, the detection of blood gas and the detection of hemoglobin and its derivatives may be performed in a single test card, so that medical resources are greatly saved, the diagnosis cost is reduced, repeatedly collecting blood is not needed, the acquisition speed of diagnosis results is accelerated, and the medical experience of a patient is remarkably improved. In addition, an optical system inside of the host has a compact structure and a small conduction loss in optical path. The blood gas and the hemoglobin and its derivatives may be accurately detected through an optical method, so that the accuracy of in-vitro diagnosis is further improved.

### BRIEF DESCRIPTION OF THE DRAWINGS

FIG. 1 is a schematic view of an out contour of an in-vitro medical diagnostic system.
FIG. 2 is a schematic side view of the in-vitro medical diagnostic system.
FIG. 3 is a schematic view of a combination of components of the in-vitro medical diagnostic system.
FIG. 4 is a schematic semi-sectional view of a removable test card.
FIG. 5 is another schematic semi-sectional view of the removable test card.
FIG. 6 is yet another schematic semi-sectional view of the removable test card.
FIG. 7 is a schematic view illustrating the connection of the removable test card and a valve control device.
FIG. 8 is a schematic control view of the valve control device.
FIG. 9 is a schematic cross-sectional view of a removable reagent pack.
FIG. 10 is a schematic view of an external structure of the removable reagent pack.
FIG. 11 is a schematic view of a body and a decoration cover of the removable reagent pack.
FIG. 12 is a schematic view of an excitation light source of the in-vitro medical diagnostic system.

### DETAILED DESCRIPTION

The present disclosure will now be further described in conjunction with the accompanying drawings and specific embodiments.

Before turning to the drawings illustrating some embodiments in detail, it is to be understood that the present disclosure is not limited to the details or methods shown in the specification or illustrated in the drawings. The purpose of professional terminology is only for illustration and should not limit the understanding of present product and corresponding methods.

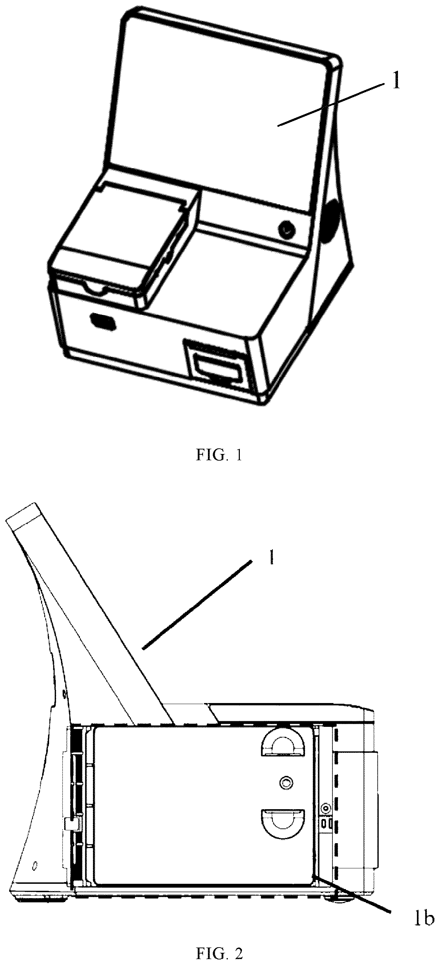

Some embodiments of the present disclosure provide an in-vitro medical diagnostic system. As shown in FIGS. 1-3, the in-vitro medical diagnostic system includes a host 1, removable test card 2, and a removable reagent pack 3. The host 1 includes a housing, a processing circuit, a power supply circuit, and an optical element. The processing circuit, the power supply circuit, and the optical element are located in the housing. The housing further includes a first area 1a configured to at least partially accommodate the removable test card 2, and a second area 1b configured to at least partially accommodate the removable reagent pack 3. The removable test card 2 is engaged with the host 1 and the removable reagent pack 3 through the first area 1a and the second area 1b, respectively, such that there is no fluid communication between the removable test card 2 and the host 1, and there is no fluid communication between the removable reagent pack 3 and the host 1.

### Removable test card

The removable test card 2 includes an external interface, a detection area, a waste liquid area, a flow path control area or a fluid path control area, and an internal flow path or internal fluid path.

The external interface includes a first interface, a second interface, and a third interface located on sides, the top, or the bottom of the test card 2. The first interface may be configured to receive a fluid sample. The second interface may be configured as an air pump inlet/outlet. The third interface may be configured to inject calibration liquid. In some embodiments, a fluid sample inlet is disposed on the first side of the test card 2. The fluid sample inlet is configured to receive a fluid sample, such as a whole blood sample that do not require hemolysis. A calibration liquid inlet and an exhaust port are on the second side of the test card 2. The calibration liquid inlet is configured to receive calibration liquid from the outside (such as the removable reagent pack 3) for calibration of various photochemical sensors inside the test card 2. The exhaust port is configured to be communicated with or fluidly coupled to the external atmosphere to maintain pressure balance in the test card 2, in order to ensure that the calibration liquid or fluid sample is able to flow into the test card 2.

The detection area refers to the area where cavities having electrical, optical, chemical sensors disposed therein for detecting various blood gas parameters and/or cavities without sensors are placed. The detection of blood gas, hemoglobin, electrolytes, and other biochemical parameters is completed in this area.

The waste liquid area refers to the area configured to store the tested fluid samples.

The internal flow path refers to the pipeline path inside the test card 2, which is configured to allow the fluid sample or calibration liquid to flow therein. The pipeline path has multiple interconnected flow paths, and the detection area, the waste liquid area, and the flow path control area are located on different flow paths.

The flow path control area refers to the area where several on-off control devices are located. The on-off control devices are configured to control an on-off state of the internal flow path, thereby achieving flow path switching, allowing the calibration liquid and fluid sample to flow into the detection area and the waste liquid area through different paths at different testing stages. The detailed switching operation will be described in detail later.

In some embodiments, the detection area, the waste liquid area, and the flow path control area are located between the first side and the second side of the test card 2.

Each functional area and the internal flow path of the test card 2 may be configured in a plurality of implementation modes, and one of the implementation modes is provided as follows.

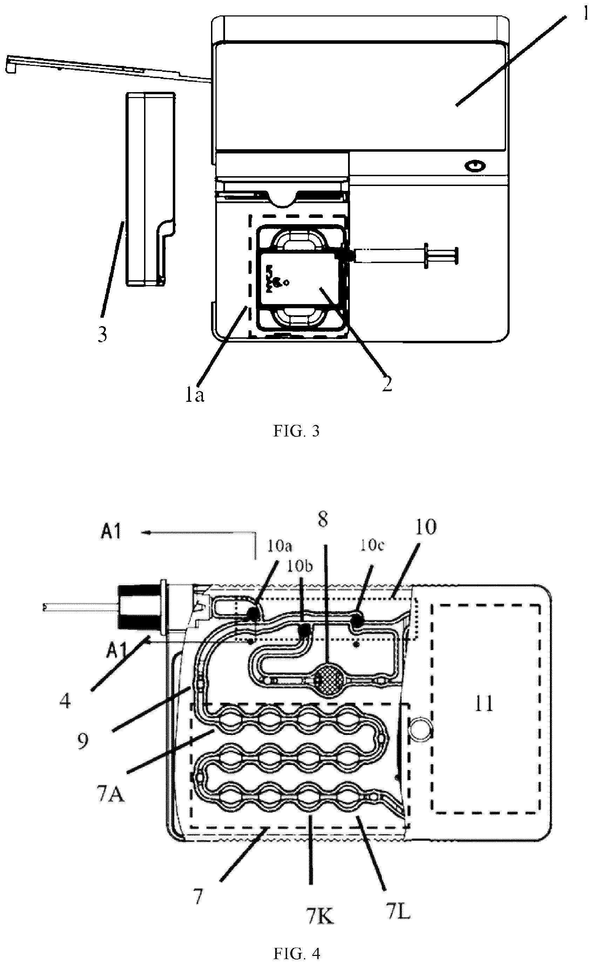

As shown in FIGS. 4-6, the removable test card 2 includes a card body, and at least a portion of the card body is transparent. The card body may include or be made of molded plastic, additional material, or a kit of materials. In order to better reflect the division of the transparent housing and internal functional areas of the card body, the semi-sectional views in FIGS. 4-6 are used to show the housing and internal functional areas of the test card 2.

The fluid sample to be detected is a blood sample. In an embodiment, the fluid sample is a whole blood sample without needing hemolysis. The detection area includes a detection area 7 in which blood gas is capable of being detected and a detection area 8 in which hemoglobin and its derivatives are capable of being detected. The waste liquid area 11 is configured to store the tested blood sample, i.e., the blood sample which has finished the test. The internal flow path 9 is divided into a main flow path or fluid path and three controllable flow paths or fluid paths. The main flow path is connected to the calibration liquid inlet 6, the detection area 7 in which the blood gas is capable of being detected and the flow path control area 10. In an embodiment, the detection area 7 in which the blood gas is capable of being detected is located between the calibration liquid inlet 6 and the flow path control area 10. The first controllable flow path is configured to control an on-off state of the flow path defined between the fluid sample inlet 4 and the main flow path. The second controllable flow path is configured to control an on-off state of the flow path defined between the main flow path and an inlet of the test area 8 in which the hemoglobin and its derivatives are capable of being detected, and an outlet of the detection area 8 in which the hemoglobin and its derivatives are capable of being detected is communicated with the waste liquid area 11. The third controllable flow path is configured to an on-off state of the flow path between the waste liquid area 11 and the main flow path. The waste liquid area 11 is fluidly connected to or communicated with the exhaust port 5. The blood gas control area and the second controllable flow path are arranged with several position monitoring points. The flow path control area 10 is provided with three valves 10a to 10c configured to control an on-off state of the internal flow path 9, and the three valves 10a to 10c are respectively configured to control the first, second and third controllable flow paths. The control mode will be described in detail in the following sections.

The detection area 7 in which the blood gas is capable of being detected has or defines twelve sensor cavities defined as 7A to 7L, the cavities are sequentially arranged on the main flow path. The cavities may be of various shapes, and the shapes of the cavities may be the same as or different from each other. However, in the flow direction of the flow path, the width of each sensor cavity is wider than the width of the flow path. Various types of sensors may be placed in the cavities. The twelve sensor cavities are arranged from far to near according to the distance from the calibration liquid inlet in the direction of the flow path, sequentially numbered 7A-7L. The first eleven sensor cavities 7A-7K are sequentially provided or arranged with different photochemical sensors, the twelfth sensor cavity 7L is used as a standby for future expansion of a detection parameter, and the detection area 8 in which the hemoglobin and its derivatives are capable of being detected is only a cavity without a sensor disposed therein.

In an embodiment, the photochemical sensors disposed in the first ten sensor cavities 7A-7J are configured to detect the blood gas parameters in the blood fluid sample, such as CO₂, O₂, pH, Na⁺, M⁺⁺, or the like.

In an embodiment, the fluid sample may be a whole blood sample, a urine sample, or other types of human body fluid samples. In this case, the sensors in the test card 2 may be configured to detect corresponding biochemical parameter indexes.

The control mode of the test card 2 is described in detail below.

Before testing the blood samples, it is necessary to calibrate all sensors in test card 2, that is, inject calibration liquid into the sensors, empty all the calibration liquid in test card 2 after calibration, and then inject a fluid sample to be detected into the test card 2. In an embodiment, the fluid sample is a blood sample that do not require hemolysis. The testing is completed in the detection area, and the corresponding parameters are read through the host of the in-vitro medical diagnosis system. After completing the diagnostic analysis, the corresponding parameters and diagnostic results are displayed on a screen of the host, such that medical personnel may timely learn about the corresponding situation.

Operations of the test card 2 are as follows:
operation S 1: placing the test card 2 into the first area 1a;
operation S2: pumping the calibration liquid into the detection area in the test card 2;
operation S3: after finishing calibration, transferring the calibration liquid in the test card 2 to the waste liquid area 11 in the test card 2;
operation S4: injecting a blood sample into the detection area to at least complete the detection of blood gas and hemoglobin and its derivatives in the detection area; and
operation S5: transferring the blood sample in the detection area to the waste liquid area 11 in the test card 2 to complete the test.

The operation S1 in some embodiments includes as follows. Before the test card 2 is engaged with the reagent pack 3 and the host 1, the test card 2 is internally dry and has no calibration liquid disposed therein. The calibration liquid is stored in the reagent pack 3 and separated from the test card 2. A detection position is the first area 1a in the host 1. When the test card 2 is placed in the first area 1a, the fluid sample in the test card 2 is detected. As shown in FIG. 3, at least six position monitoring points 11a to 11f are arranged in the test card 2 and may receive light intensity emitted by a detection light source and transmitted through the test card 2, such that whether liquid exists at the position monitoring points 11a to 11f or not may be detected and judged.

The operation S2 in some embodiments includes as follows. The third controllable flow path is in an on state and communicated with or fluidly connected to the main flow path, the first and second controllable flow paths are controllably closed / disconnected or occluded from the main flow path and in off states. The host 1 is configured to control a peristaltic pump 16 and a three-way valve 17 in the reagent pack 3, switch the three-way valve 17 to a calibration liquid pipeline 19, pump the calibrating liquid (i.e., a standard sample liquid with known composition and concentration) in a calibration liquid reservoir 15 into the test card 2 through a connecting member 20 inserted into the calibration liquid inlet of the test card 2. When it is detected that liquid exists at the position monitoring point 11a, the host 1 is configured to control the peristaltic pump 16 in the reagent pack 3 to stop operating, and at the moment, the sensor cavities 4A to 4K are filled with the calibration liquid. The host 1 may start calibrating the sensors (i.e., reading the readings of the standard sample measured by the liquid sensors), and the host 1 may finish calibrating after reading detection values of the sensors.

The operation S3 in some embodiments includes as follows. The third controllable flow path is kept communicated with or fluidly connected to the main flow path, and the first and second controllable flow paths are kept closed and occluded from the main flow path. The host 1 is configured to control the three-way valve 17 in the reagent pack 3 to be switched to an air channel. The peristaltic pump 16 is controlled and enabled to pump air into the test card 2 through the connecting member 20 inserted into the calibration liquid inlet of the test card 2. Since the waste liquid area 11 of the test card 2 is communicated with or fluidly connected to outside air through the exhaust port 5, with the pumping of the air, the calibration liquid may continuously move to the waste liquid area 11 in the first and third flow paths until all the calibration liquid enters the waste liquid area 11.

The operation S4 includes two stages, i.e., a first stage and a second stage as follows.

The blood gas detection is performed at the first stage. In some embodiments, at the first stage, the first controllable flow path is communicated with or fluidly connected to the main flow path, the second and third controllable flow paths are closed and occluded from the main flow path, and the blood gas detection is performed in this case. The blood sample is injected from the sample inlet 4 of the test card 2, without needing hemolysis, or the host 1 is configured to control the peristaltic pump 16 to rotate reversely such that the air in the test card 2 is extracted and negative pressure is generated in the test card 2 to suck blood at the sample inlet 4 into the inner side of the test card 2. Thus, after it is determined, via the position monitoring points 12a to 12f, that the blood sample to be detected completely enters the sensor cavities 4A to 4L, the blood sample to be detected is stopped injecting into the test card 2, or the host 1 is configured to control the peristaltic pump 16 to stop pumping air, and the blood sample is detected or tested by using the sensor cavities 4A to 4L. In an embodiment, the photochemical sensors are arranged in the sensor cavities 4A to 4K, the sensor cavity 4L is used as standby, or other types of sensors may be placed in the sensor cavity 4L. A basic detection principle of the photochemical sensor uses a photochemical method, which utilizes organic dyes to emit fluorescence with different wavelengths from the irradiation light under specific wavelengths of light and influenced by factors such as concentrations of O₂ and CO₂, and pH. The fluorescence is transmitted to a detector to detect a fluorescence signal of the fluorescence and perform quantitative analysis, thereby being able to detect the concentration of O₂, the concentration of CO₂, and a pH value.

It may enter the second stage after completing the blood gas detection, that is, hemoglobin and its derivatives are detected at the second stage. The first controllable flow path is kept communicated with or fluidly connected to the main flow path, the third controllable flow path is kept closed and occluded from the main flow path, and the second controllable flow path is opened and switched to be communicated with or fluidly connected to the main flow path. The host 1 is configured to control the peristaltic pump 16 to rotate forward and pump air into the test card 2, such that the blood in the blood gas detection area is transferred into the detection area 8 in which the hemoglobin and its derivatives are capable of being detected through the second controllable flow path. Then the host 1 may be configured to control the peristaltic pump 16 to stop pumping air into the test card 2. The light emitted from a first light source is used to irradiate the detection area 8 in which the hemoglobin and its derivatives are capable of being detected from one side, and the transmitted light from the other side of the detection area 8 in which the hemoglobin and its derivatives are capable of being detected is received, and whether hemoglobin and its derivative are present in the blood sample or not is determined according to the detection principle of colorimetric method.

The operation S5 in some embodiments includes as follows. After completing the detection of hemoglobin and its derivative, the host 1 is configured to control the peristaltic pump 16 to rotate forward and pump air into the test card 2 to push the blood sample in the detection area 8 for hemoglobin and its derivative into the waste liquid area 11. After the blood sample enters the waste liquid area 11, the host 1 is configured to control the peristaltic pump 16 to stop rotating, and the test is finished.

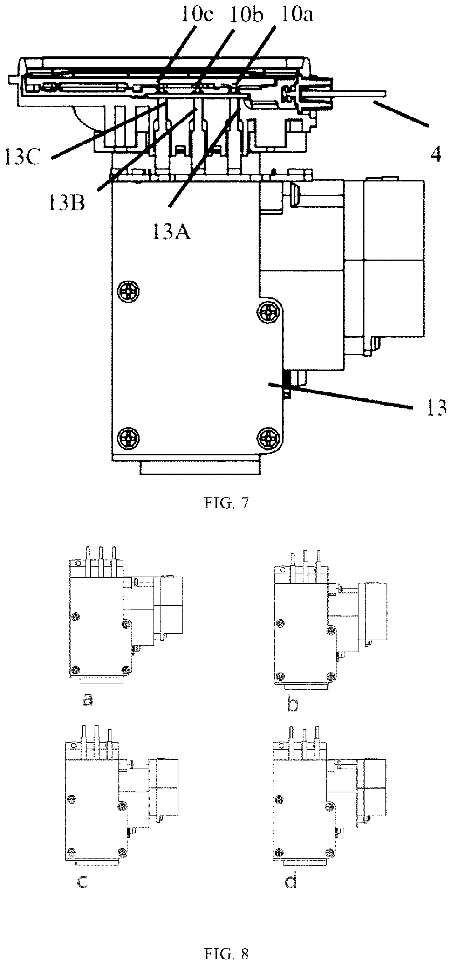

In the above operations, the control of the first, second, and third controllable flow paths is realized as shown in FIGS. 7 to 8.

The three valves 10a to 10c are disposed inside of the flow path control area 10. The valves 10a to 10c are respectively configured to control the on-off states of the first, second, and third controllable flow paths, and the on-off states of the valves 10a to 10c are respectively driven or controlled by control mechanisms 13A to 13C in a valve control device 13. When one of the control mechanisms 13A to 13C moves downwards, one corresponding valve in the valves 10a to 10c is switched on, and the corresponding controllable flow path is in the on state and communicated with or fluidly connected to the main flow path. Otherwise, when one of the control mechanisms 13A to 13C moves upwards to the end, one corresponding valve in the valves 10a to 10c is closed or switched off, and the corresponding controllable flow path is occluded from the main flow path.

In some embodiments, in the operation S1, the valves 10a to 10c are all in off states and switched off. The positions of the control mechanisms are shown in FIG. 8a, that is, the first, second, and third controllable flow paths are all disconnected or occluded from the main flow path.

In the operations S2 and S3, the valves 10a to 10c are in off states and switched off, while the valve 10c is in an on state and switched on. The positions of the control mechanisms are shown in FIG. 8b. The main flow path is communicated with or fluidly connected to the waste liquid area 11, such that the calibration liquid may be pumped into the test card 2. After calibration is completed, air is pumped into the test card 2, such that the calibration liquid may enter the waste liquid area 11.

At the first stage of the operation S4, the valve 10a is controlled to be switched on, the valves 10b to 10c are switched off. The positions of all the control mechanisms are shown in FIG. 8c. The main flow path is communicated with or fluidly connected to the sample inlet 4, such that the blood sample may be injected into the test card 2, and the sample blood reaches the detection area 7 in which the blood gas is capable of being detected for blood gas detection.

At the second stage of operation S4, that is, after the blood gas detection is completed, the valve 10b is controlled to be switched on, and the valves 10a and 10c are switched off. The positions of the control mechanisms are shown in FIG. 8d. The main flow path is communicated with or fluidly connected to the detection area 8 for hemoglobin and its derivatives, such that the blood sample may enter the detection area 8 for hemoglobin and its derivatives, and the detection of the hemoglobin and its derivatives is performed.

After the detection is completed, the blood sample is transferred into waste liquid area 11.

The valves 10a to 10c may be switched off based on a fixed timing sequence, or a switching-off sequence of the valves 10a to 10c may be customized and logically programmed according to needs of an operator.

In an embodiment, the widths of the first, second, and third flow paths may be set to be different from each other. In an embodiment, the width of the second flow path is greater than the width of the first flow path. The thickness of the sensor cavity of the test card 2 in the detection area 7 in which the blood gas is capable of being detected is different from the thickness of the cavity in the detection area 8 for hemoglobin and its derivatives, so as to meet requirements of different detection methods for the blood gas photochemical detection of the whole blood sample and the detection of hemoglobin and its derivatives.

In an embodiment, the test card 2 is configured to be discarded after being sued. In some embodiments, the test card 2 may be configured to be recycled to test more than one fluid sample.

Furthermore, an electrochemical sensor may be used in the test card 2 to detect blood gas, hemoglobin and its derivatives. The electrochemical detection technology is mature, which is not required to be described in detail.

### Reagent pack

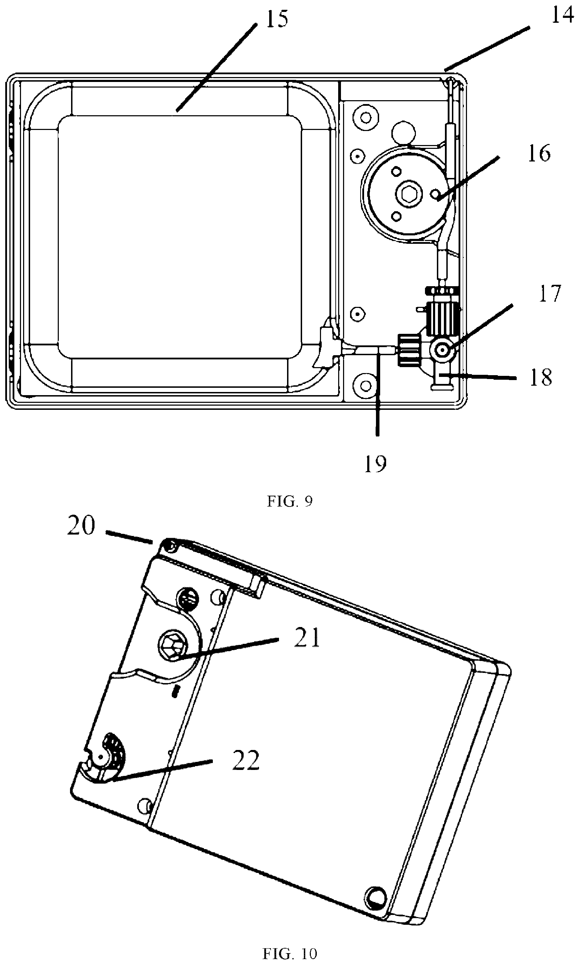

As shown in FIG. 9, FIG. 9 is a schematic cross-sectional view of the reagent pack 3. The reagent pack 3 may be disposable and removable. The reagent pack 3 includes a housing 14, the calibration liquid reservoir 15, the peristaltic pump 16, the three-way valve 17, an air pipeline 18, a calibration liquid pipeline 19, a connecting member 20, a pump interface 21, and a valve connecting member 22. The calibration liquid reservoir 15, the peristaltic pump 16, the three-way valve 17, the air pipeline 18, and the calibration liquid pipeline 19 are located in the housing 14. The connecting member 20 is inserted into the calibration liquid inlet 6 of the test card 2. The pump interface 21 is connected to a rotating shaft of a stepper motor of the host 1. The stepper motor is configured to drive the peristaltic pump 16 of the reagent pack 3 to rotate forwardly or reversely through the pump interface 21. The host 1 may be configured to control the three-way valve 17 of the reagent pack 3 to switch through the valve connecting member 22, such that the pump interface 21 is communicated with or fluidly connected to the air pipeline 18 or the calibration liquid pipeline 19. The calibration liquid reservoir 15 is configured to store the calibration liquid and is configured to be communicated with or fluidly connected to the three-way valve 17 through the calibration liquid pipeline 19.

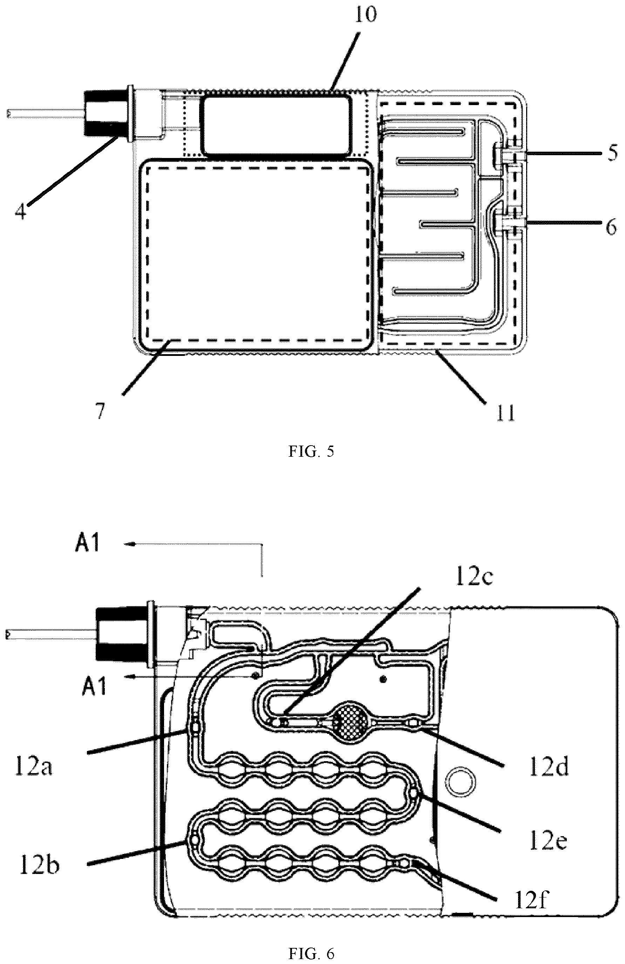

In some embodiments, the housing 14 of the reagent pack 3 may include or be made of plastic. In some embodiments, the housing 14 may also be made of other materials or a kit of materials. The reagent pack 3 may further include a decoration cover. As shown in FIG. 6, the decoration cover 23 of the reagent pack 3 is connected to a front part of the housing 14 of the reagent pack 3. When the reagent pack 3 is not in use (i.e., not engaged with the host 1), the decoration cover 23 of the reagent pack 3 may protect the housing 14 of the reagent pack 3. The calibration liquid reservoir 15 may be a soft elastic fluid bag filled with unused calibration liquid.

Before the test card 2 and the reagent pack 3 are respectively engaged with the host 1, the interior of the test card 2 is dry and has no calibration liquid, and all the calibration liquid is stored in the reagent pack 3 and is separated from the test card 2.

When the test card 2 is placed in the first area 1a, the connecting member 20 on the reagent pack 3 is inserted into the calibration liquid inlet 6 of the test card 2. In an embodiment, the connecting member 20 is a tubular steel needle, and a sealing ring, such as a rubber sealing ring, is arranged on a periphery of the tubular steel needle, in order to ensure the sealing performance at an inserting position of the tubular steel needle and the calibration liquid inlet 6 of the test card 2. After the calibration liquid is pumped into the test card 2, the calibration liquid will not be leaked from the calibration liquid inlet 6. A controller in the host 1 may be configured to control the rotating shaft of the stepper motor to output power, and the peristaltic pump 16 in the reagent pack 3 is controlled to rotate forwardly or reversely through the pump interface 21, such that the test card 2 may complete calibration and fluid sample detection. The working principle is described in detail below.

When the test card 2 and the reagent pack 3 are respectively arranged in the first area 1a and the second area 1b of the host 1, the working principle of the reagent pack 3 is as follows.

When the calibration liquid in the reagent pack 3 needs to be output, such as, at the calibration stage of the test card 2 in the operation S2, the three-way valve 17 in the reagent pack 3 is switched to enable the calibration liquid pipeline 19 to be communicated with or fluidly connected to the connecting member 20. The peristaltic pump 16 rotates forwardly, such that the reagent pack 3 may output the calibration liquid outwards. After the calibration liquid enters the test card 2, the photochemical sensors in the test card 2 may be calibrated.

When air needs to be pumped to the outside, for example, when the calibration liquid and the blood sample which has been tested are transferred to the waste liquid area 11 of the test card 2 in the operation S3 and the operation S5, or when the blood sample is transferred to the test area 8 in which the hemoglobin and its derivatives are capable of being detected after the blood gas detection is completed at the second stage of operation S4, the three-way valve 17 in the reagent pack 3 is switched to enable the air pipeline 18 to be communicated with or fluidly connected to the connecting member 20. The peristaltic pump 16 rotates forwardly, such that the reagent pack 3 may pump air into the test card 2, and the blood sample in the test card 2 may flow in the flow path communicated in the test card 2 with the pumping of the air.

When air needs to be sucked into the reagent pack 3, for example, when the blood sample is sucked into the detection area 7 in which the blood gas is capable of being detected in the test card 2 at the first stage of the operation S4, the three-way valve 17 in the reagent pack 3 is switched to enable the air pipeline 18 to be communicated with or fluidly connected to the connecting member 20. The peristaltic pump 16 rotates reversely such that the air in the test card 2 is sucked into the reagent pack 3, and the blood sample enters the flow path in the test card 2 under the action of air pressure, thereby performing the blood gas detection.

### Host

As previously described, the host 1 includes the housing, the processing circuit, the power supply circuit, and the optical element, which are located in the housing, respectively. The housing may include or be made of plastic or any other material suitable for use in the present disclosure. The housing includes the first area 1a configured to at least partially accommodate the removable test card 2 and the second area 1b configured to at least partially accommodate the removable reagent pack 3. The removable test card 2 is engaged with the host 1 and the removable reagent pack 3 through the first area 1a and the second area 1b, respectively, thereby performing blood gas detection, hemoglobin and its derivatives detection, or other biochemical parameter detection. Only mechanical transmission connection exists between the test card 2 and the host 1, and there is no flow path connection between the test card 2 and the host 1. Only mechanical transmission connection exists between the reagent pack 3 and the host 1, and there is no flow path connection between the reagent pack 3 and the host 1. The test card 2 is connected to the reagent pack 3 through the calibration liquid inlet 6, and the flowing of the calibration liquid and the air are achieved. Due to above design, a flow path system does not exist in the host 1, and flow paths flowing between the host 1 and the test card 2, and between the host 1 and the reagent pack 3 are not needed.

In an embodiment, the first area 1a of the housing includes a test slot configured to accommodate the test card 2. A syringe containing the fluid sample, such as the blood sample, is configured to be communicated with or fluidly connected to the sample inlet 4 of the test card 2. The host 1 is configured to test the fluid sample and report the result to the user through an output unit. For example, host 1 may include a display serving as the output unit. However, in this embodiment or other embodiments, the diagnostic result may also or alternatively be reported to the user by other output units, including an audio output unit, a data communication output unit, or a print output unit, and so on.

In an embodiment, once the fluid sample is tested, the test card 2 may be removed from the host 1. The host 1 may include an ejection button, once the test is complete, the user may press the ejection button to eject the test card 2 from the test slot. When the test cycle is completed, the host 1 may also be configured to automatically eject the test card 2. In an embodiment, the host 1 may be portable.

In an embodiment, the diagnostic result may be displayed on the display. The processing circuit of the host 1 may enable the display to show or display information related to a particular application. The display may be a unidirectional screen configured to display the output to the user. Or the display may be a touch screen configured to receive and respond to a touch input of the user. In an embodiment, the diagnostic device further includes a printing slot configured to receive a paper output by a printer housed within the host 1.

In an embodiment, as shown in FIG. 3, the second area 1b of the host 1 includes a door for a reagent pack, and the door is configured to be opened from a side of the host 1. An opening behind the door and the door are configured to accommodate the reagent pack 3. The door may be opened by a latch. In other embodiments, the door may also be opened by other mechanisms. The door may also be located at other positions, for example, the door may be located on either side, the back, the front, or the top of the host 1. The latch is positioned adjacent to the door. The power supply interface for supplying power to the pump and the power supply interface for supplying power to the valve components are set outside of the reagent pack.

In an embodiment, except for the connecting member 20 of the reagent pack 3 being directly connected to the calibration fluid inlet 6 of the test card 2, the reagent pack 3 may also be engaged with the host 1 through the following methods. The connecting member 20 of the reagent pack 3 is connected to a host sample inlet above one side of the host 1, and the host sample inlet is further communicated with or fluidly connected to the calibration liquid inlet 6 of test card 2 to ensure that the calibration liquid may flow into the test card 2. The calibration liquid reservoir 15 of the reagent pack 3 includes an engaging groove, and the engaging groove is configured as a fixing device of the reagent pack 3 configured to be engaged with one side of the host 1, in order to fix the reagent pack 3 in the second area 1b of host 1. The valve connecting member 22 of the reagent pack 3 is connected to the on-off valve control device of the reagent pack 3 on one side of the host 1, thereby achieving on-off control of the three-way valve 17 of the reagent pack 3 by the host 1. In addition, the pump interface 21 of the reagent pack 3 is connected to a control device for the peristaltic pump, such as the output shaft of the stepper motor, on one side of the host 1, such that the host 1 may be configured to control the peristaltic pump 16 in the reagent pack 3.

In an embodiment, the host 1 may include one or more ports configured to accommodate a cable or other connection mechanism. The port may be configured to connect the host 1 to other pieces of the system (such as via a communication network) or to upload information or download information to the host 1. The host 1 may also be configured to exchange data wirelessly, including through Wi Fi (wireless internet technology), another wireless internet connection, or any other wireless information exchange. The host 1 may also include a speaker configured to transmit noise or sound response to the user. The host 1 may also include a handle allow the user to hold the host 1. The handle rotates between two positions depending on whether it is used or not. In some embodiments, the host 1 may also include a support leg configured to allow the host 1 to be placed on a desktop or other surface. In an embodiment, the host 1 may also include a barcode scanner installed on the side of the host 1. The barcode scanner is configured to scan barcodes on the test card 2, calibration liquid reservoir 15, or any other items that have a scannable barcode and that are used on the host 1. The barcode scanner may also be configured to scan barcode label that represents an identity of a patient or an identity of an operator. In an embodiment, the barcode scanner emits a beam of light covering the barcode. When the barcode is successfully scanned, the host 1 emits a beep sound and the beam is automatically turned off. When the barcode is not successfully scanned, the host 1 alerts the user by making noise or on the display through another output unit. In embodiments, the barcode scanner is a one-dimensional barcode scanner. In other embodiments, the barcode scanner is a two-dimensional scanner.

According to an embodiment, the processing circuit of the host 1 includes an analog-to-digital converter and an analog control board. The analog-to-digital converter is configured to process an analog signal from the photochemical sensor, and transmit the processed digital signal to the analog control board. When the analog control board herein and in the drawings is designated as "analog control board", the analog control board may include digital processing. Furthermore, the analog control board may utilize the digital-to-analog converter to convert a digital output (turning on/turning off modulated signal) to the analog signal (e.g., for the photochemical sensor).

The processing circuit and power supply circuit in the host 1 may be used as independent printed circuit boards (PCB), integrated on the same PCB, or combined with integration and distribution. The processing circuit and the power supply circuit may include discrete components and/or integrated circuits. For example, the power supply circuit may include all discrete electronic components. The processing circuit may include one or more processors. The processor may be operated in multiple ways as a general-purpose processor, an application specific integrated circuit (ASIC), one or more field programmable gate arrays (FPGA), a set of processing components, or other suitable electronic processing components. The processing circuit may also include one or more memories. The memory may be one or more devices configured to store data and/or computer code. The memory may be or include a non-transitory volatile memory and/or a non-volatile memory. The memory may include a database component, an object code component, a script component, or any other type of information structure configured to support various activities and the information structure described herein. The memory may be transmissibly connected to the processor and includes computer code modules for executing one or more programs described herein.

The optical elements of the host 1 are now described in detail as previously described. In embodiments of the present disclosure, the blood gas parameters in the blood sample are measured by the photochemical sensors, and hemoglobin and its derivatives are detected by using optical methods, such as colorimetry without the photochemical sensors. Thus, the optical element is needed to provide the light source, excite the photochemical sensor, transmit the optical signals, and the like.

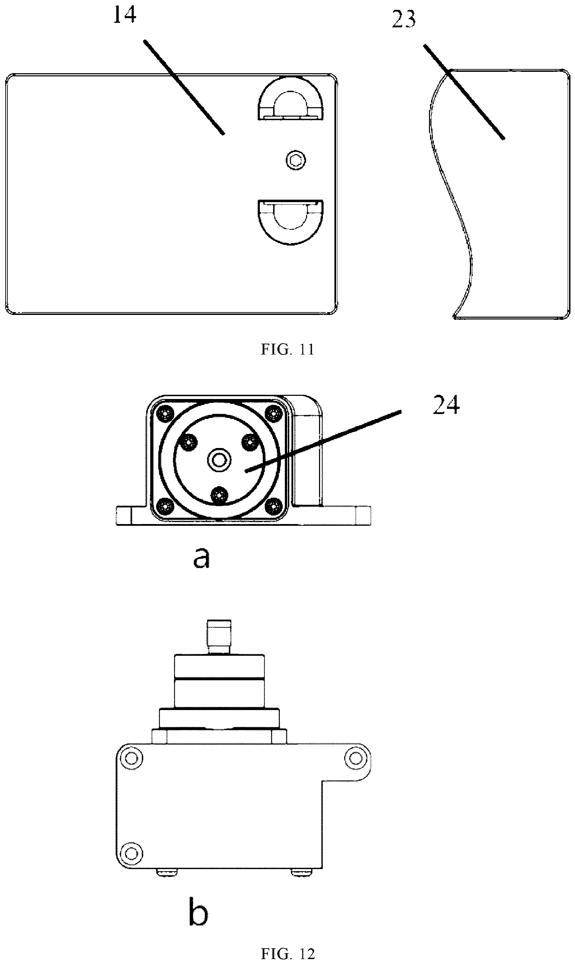

In some embodiments, the optical element of the host 1 includes two parts. A first part of the optical element is arranged in the host 1, and the first part includes a first light source configured for the detection of the hemoglobin and its derivatives and a second light source configured for the detection of a position of the fluid in the test card 2. The first light source is configured to emit a detection beam for the detection of the hemoglobin and its derivatives of the blood sample, and the second light source is configured to detect the presence or absence of fluid in each position monitoring point of the test card 2, respectively. A second part of the optical element is arranged in the host 1 and equipped with a light source 24 for optically detecting the blood gas component of the blood sample, as shown in FIG. 12. In some embodiments, the light source 24 is an excitation light source 24 configured to emit an excitation beam to excite the photochemical sensor in the test card 2, and the excitation light source is located within the host 1 and is lower in height or located at a lower level than the first area 1a.

In an embodiment, the diagnostic device includes the valve control mechanism configured to control the valves of the test card 2.

The terms "generally", "about", "substantially", and similar terms used here have a broad meaning consistent with the generally accepted usage recognized by those skilled in the art, and the subject of the present disclosure belongs to the generally accepted usage. Those skilled in the art who review the present disclosure should understand that these terms are intended to allow descriptions of certain features described and claimed, without limiting the scope of these features to a set precise numerical range. Therefore, these terms should be interpreted as non-substantive or incoherent modifications or modifications of the subject matter described and claimed priority should be considered within the scope of the present disclosure and explained by the accompanying claims. It should be noted that the term "exemplary" used in this disclosure to describe different embodiments refers to possible examples, illustrations representing and/or possible embodiments (this term does not imply that such embodiments must be extraordinary examples or highest-level examples). The terms "coupled" and "connected" and their analogues used in the present disclosure refer to the direct or indirect combination of two components with each other. This combination may be static (such as permanent) or movable (such as removable or releasable). This combination may be achieved by combining two components or integrating the two components with any additional intermediate components into a single entity, or by combining two components or combining two components attached to each other with any additional intermediate components. The structure and arrangement of the system and the method for providing the in-vitro medical diagnostic device as shown in various embodiments are just illustrated.

Only some embodiments have been described in detail in present disclosure, but it will be readily appreciated by those skilled in the art with reference to this disclosure, there may be many modifications (e.g., variations in size, dimension, structure, shape, and scale of various elements, parameter values, mounting arrangements, use of materials, color, changes in orientation, and so on.) without substantially departing from the novel teachings and advantages of the subject matter disclosed herein. For example, the components shown as a whole may include multiple parts or components, and the position of the components may be reversed or otherwise changed, and the property, quantity, or position of discrete components may be changed or varied. All such modifications are intended to be included in the scope of the present disclosure as defined in the appended claims. The order or sequence of any process or method step may be changed or reordered according to alternative embodiments. The design, operating conditions, and arrangement of various embodiments may be replaced, modified, altered, and omitted without departing from the scope of the present disclosure.

The diagnostic device is generally shown to include the processing circuit including the memory. The processing circuit may include the processor. The processor may be operated as a general-purpose processor, a specialized integrated circuit (ASIC), one or more field programmable gate arrays (FPGA), a set of processing components, or other suitable electronic processing components. The memory may be one or more devices configured to store data and/or computer code (e.g., RAM, ROM, flash memory, hard disk memory, or the like), to complete and/or implement various programs described in present disclosure. The memory may be or include a non-transitory volatile memory and/or a non-volatile memory. The memory may include the database component, the object code component, the script component, or any other type of information structure configured to support various activities and the information structure described herein. The memory may be transmissibly connected to the processor and includes computer code modules for executing one or more programs described herein. The foregoing is merely some embodiments of the present disclosure, without limiting the present disclosure. Any modifications, equivalent substitutions, and modifications made within the spirit and principles of the disclosure should be included within the scope of the present disclosure.

## Claims

**1.** An in-vitro medical diagnostic device, **characterized by** comprising:
a housing, wherein the housing comprises:
a first mounting area, configured to dispose a removable reagent pack;
a second mounting area, configured to dispose a removable test card;
an optical system, disposed in the housing and configured for in-vitro diagnosis; and
a power drive system, disposed in the housing and configured to control at least pipelines in the removable test card to be fluidly connected to each other or occluded from each other, and/or control liquid flow in the removable reagent pack, wherein the optical system is at least configured to detect a blood gas, a hemoglobin and/or an electrolyte in the removable test card.

**2.** The in-vitro medical diagnostic device as claimed in claim 1, further comprising a positioning connection area configured to connect an interface of the removable reagent pack and an interface of the removable test card.

**3.** The in-vitro medical diagnostic device as claimed in claim 1, wherein the optical system has one or more light source systems.

**4.** The in-vitro medical diagnostic device as claimed in claim 3, wherein at least one first light source system configured to activate a photochemical sensor in the removable test card is disposed below the second mounting area.

**5.** The in-vitro medical diagnostic device as claimed in claim 3, wherein the first light source system is configured to emit an excitation light, and the excitation light is configured to pass through a first optical path and enters one end of an optical fiber bundle, and the other end of the optical fiber bundle is provided with an optical element configured to focus and illuminate the photochemical sensor in the removable test card.

**6.** The in-vitro medical diagnostic device as claimed in claim 5, wherein the first optical path comprises at least a first lens, a first filter, and a first beam splitter.

**7.** The in-vitro medical diagnostic device as claimed in claim 5, wherein the fiber optic bundle is configured to receive fluorescence and/or reflected light generated in the removable test card, and transmit the fluorescence and/or the reflected light to an optical detection element through a second optical path, and the second optical path is at least partially different from the first optical path.

**8.** The in-vitro medical diagnostic device as claimed in claim 3, the optical system further comprises at least one second light source system disposed above the second mounting area and configured to detect hemoglobin and its derivatives and/or detect a flow position of internal liquid in the removable test card.

**9.** The in-vitro medical diagnostic device as claimed in claim 4, wherein an excitation light emitted from the first light source has a central wavelength of 450 nm to 470 nm.

**10.** The in-vitro medical diagnostic device as claimed in claim 8, wherein a central wavelength of the fluorescent and/or reflected light ranges from 520 nm to 650 nm.

**11.** The in-vitro medical diagnostic device as claimed in claim 1, wherein the optical system comprises at least two light sources located on different sides of the second mounting area.

**12.** The in-vitro medical diagnostic device as claimed in claim 1, wherein the power drive system further comprises a first power component configured to control the pipelines in the removable test card to be fluidly connected to each other or occluded from each other. 13. The in-vitro medical diagnostic device as claimed in claim 1, wherein the power drive system further comprises a second power component configured to control switching of the pipelines and liquid flow in the removable reagent pack.

**14.** The in-vitro medical diagnostic device as claimed in claim 13, wherein the second power component comprises a first motor configured to control switching of a three-way valve in the removable reagent pack and a second motor configured to control rotation of a peristaltic pump in the removable reagent pack.

**15.** The in-vitro medical diagnostic device as claimed in claim 1, wherein the power drive system further comprises a third power component configured to lock and unlock the removable test card.

**16.** The in-vitro medical diagnostic device as claimed in claim 1, wherein the power drive system is configured to outputs power in one or more modes of mechanical transmission, electricity, magnetism, or light.

**17.** The in-vitro medical diagnostic device as claimed in claim 1, further comprising one or more of: a display element configured to display diagnostic results, a printing element configured to output the diagnostic results, bar code/two-dimensional code reading elements, a battery, a UPS power supply.

**18.** An in-vitro medical diagnosis system, **characterized by** comprising:
a blood gas analyzer host;
a removable test card;
a removable reagent pack, wherein an interface of the removable reagent pack is fluidly connected to an interface of the removable test card (2); and
an in-vitro medical diagnostic device configured to provide power to the removable reagent pack and control pipelines of the removable test card to be fluidly connected to each other or occluded from each other, to complete calibration, sample detection, and emptying.

**19.** The in-vitro medical diagnosis system as claimed in claim 18, wherein the removable test card comprises at least a detection area in which blood gas is capable of being detected and a detection area in which hemoglobin and its derivatives are capable of being detected.

**20.** The in-vitro medical diagnosis system as claimed in claim 19, wherein a detection in the detection area in which hemoglobin and its derivatives are capable of being detected is implemented by colorimetric, electrochemical, or hemolytic method.

**21.** The in-vitro medical diagnosis system as claimed in claim 18, wherein the removable reagent pack comprises at least one liquid storage device and its output pipelines, at least one transport control device configured to control at least flow directions of pipelines inside of the removable reagent pack, and at least one positioning mechanism configured to enable the transport control device to be driven by the in-vitro medical diagnostic device.

**22.** The in-vitro medical diagnosis system as claimed in claim 19, wherein pipelines between the detection area in which blood gas is capable of being detected and the detection area in which hemoglobin and its derivatives are capable of being detected are configured to be fluidly connected to each other or occluded from each other.

**23.** The in-vitro medical diagnosis system as claimed in claim 18, wherein the in-vitro medical diagnostic device is configured to control the pipelines of the removable test card to be fluidly connected to each other or occluded from each other, so that at least blood gas detection and hemoglobin detection is performed on the same blood sample or on different blood samples.

**24.** The in-vitro medical diagnosis system as claimed in claim 18, wherein the removable reagent pack at least comprises calibration liquid required for calibration of the removable test card one or more times.

**25.** The in-vitro medical diagnosis system as claimed in claim 18, wherein an elastic material and/or a valve is disposed inside the removable test card or on a surface of the removable test card, and the elastic material and/or the valve is configured to allow the pipelines to be fluidly connected to each other or occluded from each other at a corresponding position of the removable test card.

**26.** The in-vitro medical diagnosis system as claimed in claim 18, wherein the removable test card comprises at least three external interfaces; and the at least three external interfaces comprise a first interface configured to inject a calibration liquid, a second interface configured to inject a sample, and a third interface configured to be fluidly connected to a waste liquid area of the removable test card.

**27.** The in-vitro medical diagnosis system as claimed in claim 18, wherein the removable test card comprises at least three pipeline control components.

**28.** The in-vitro medical diagnosis system as claimed in claim 27, wherein the at least three pipeline control components comprise a first pipeline control component, a second pipeline control component, and a third pipeline control component; wherein the first pipeline control component is configured to control pipelines between the detection area in which blood gas is capable of being detected and the detection area in which hemoglobin and its derivatives are capable of being detected to be fluidly connected to each other or occluded from each other;
the second pipeline control component is configured to enable a flow path inside of the removable test card to be fluidly connected to a sample inlet of the removable test card, or control the flow path inside of the removable test card to be occluded from the sample inlet of the removable test card; and
the third pipeline control component is configured to enable the waste liquid area of the removable test card to be fluidly connected to the flow path inside of the removable test card, or control the waste liquid area of the removable test card to be occluded from the flow path inside of the removable test card.

**29.** The in-vitro medical diagnosis system as claimed in claim 28, wherein the in-vitro medical diagnostic device is electrically connected to the removable test card.

**30.** The in-vitro medical diagnosis system as claimed in claim 29, wherein the in-vitro medical diagnostic device is electrically connected to the removable test card and configured to supply power to an electrochemical sensor in the removable test card.
